# EUROPEAN PATENT SPECIFICATION

(11) **EP 4 223 749 B1**
(45) Date of publication and mention of the grant of the patent: **06.08.2025**
(21) Application number: 21890469.6
(22) Date of filing: 21.01.2021
(51) Int. Cl.: C07D 311/30

(54) **PREPARATION METHOD FOR CANNFLAVIN COMPOUNDS**
VERFAHREN ZUR HERSTELLUNG VON CANNFLAVINVERBINDUNGEN
PROCÉDÉ DE PRÉPARATION DE COMPOSÉS DE CANNFLAVINE

(30) Priority: 12.11.2020 CN 202011262162
(43) Date of publication of application: 09.08.2023
(73) Proprietor: Deyi Pharmaceutical Ltd., Kunming, Yunnan 650100 (CN)
(72) Inventor: LUO, Junlu, Kunming, Yunnan 650100 (CN); MOU, Hongtao, Kunming, Yunnan 650100 (CN); DU, Yesong, Kunming, Yunnan 650100 (CN); TAN, Xin, Kunming, Yunnan 650100 (CN); WANG, Shubin, Kunming, Yunnan 650100 (CN); ZHANG, Pingping, Kunming, Yunnan 650100 (CN); LAN, Lan, Kunming, Yunnan 650100 (CN)
(74) Representative: HGF
(86) International application number: PCT/CN2021/073145
(87) International publication number: WO 2022/099931

(56) References cited:
- WO-A2-2017/027136
- WO-A2-2017/091837
- CN-A- 102 018 698
- CN-A- 102 558 164
- CN-A- 104 031 016
- CN-A- 107 686 472
- CN-A- 109 369 591
- ZHAO YUAN ET AL: "Facile synthesis of acacetin and its derivatives", BIOORGANIC & MEDICINAL CHEMISTRY LETTERS, vol. 26, no. 15, 9 June 2016 (2016-06-09), Amsterdam NL, pages 3577 - 3580, XP093137907, ISSN: 0960-894X, DOI: 10.1016/j.bmcl.2016.06.018
- ZHAO YUAN ET AL: "Supplementary Information", 9 June 2016 (2016-06-09), XP093138181, Retrieved from the Internet <URL:https://ars.els-cdn.com/content/image/1-s2.0-S0960894X16306291-mmc1.pdf> [retrieved on 20240306]
- MAURICIO OSORIO ET AL: "Synthesis and DPPH Radical Scavenging Activity of Prenylated Phenol Derivatives", MOLECULES, vol. 17, no. 12, 6 January 2012 (2012-01-06), pages 556 - 570, XP055344810, DOI: 10.3390/molecules17010556
- SALEM, M.M.;CAPERS, J.;RITO, S.;WERBOVETZ, K.A.: "Antiparasitic activity of C-geranyl flavonoids from Mimulus bigelovii", PHYTOTHERAPY RESEARCH, 1 June 2012 (2012-06-01), UK , pages 1246 - 1249, XP018505208, ISSN: 0951-418x, DOI: 10.1002/ptr.3404
- LUIS ESPINOZA; LAUTARO TABORGA; KATY DÍAZ; ANDRÉS OLEA; HUGO PEÑA-CORTÉS: "Synthesis of Linear Geranylphenols and Their Effect on Mycelial Growth of Plant Pathogen Botrytis cinerea", MOLECULES, vol. 19, no. 2, 27 January 2014 (2014-01-27), pages 1512 - 1526, XP055344808, DOI: 10.3390/molecules19021512
- THÉVENIN MARION, THORET SYLVIANE, DUBOIS JOËLLE: "First Total Synthesis of Original Chalcone-Flavone Dimers as Cissampeloflavone Analogues", EUROPEAN JOURNAL OF ORGANIC CHEMISTRY, vol. 2018, no. 42, 15 November 2018 (2018-11-15), DE , pages 5843 - 5852, XP055895535, ISSN: 1434-193X, DOI: 10.1002/ejoc.201800338

## Description

### TECHNICAL FIELD

The present invention relates to the field of chemical synthesis, and particularly to a preparation method for a cannflavin compound, especially cannflavin A and/or cannflavin C.

### BACKGROUND

Among over 200 biologically-active compounds of cannabis sativa, cannabinoids and terpenes have been of greatest concern. However, there is another important compound in cannabis sativa, that is cannflavin compounds which account for 10% of these known compounds, of which about 20 types are known to be present in cannabis sativa, and these flavonoids have no psychoactive properties. Studies proved that Cannflavin A has anti-inflammatory effects as early as 1985, and cannflavin C was discovered in 2008. In contrast to other analgesic agents, cannflavin A and cannflavin C do not induce a risk of drug dependence and addiction in patients.

The flavonoid backbone and geranyl group on the backbone of cannflavin A and cannflavin C can improve the lipophilicity of molecules, thereby enhancing cell uptake of the molecules and their accumulation in organisms, and promote the interaction between the molecules and cells, for example, the molecules participate in the transduction pathways of membrane-bound enzymes and receptor cell signals.

Moreau et al. reported in Flavonoid Derivative of Cannabis Demonstrates Therapeutic Potential in Preclinical Models of Metastatic Pancreatic Cancer. ([J]. Front. Oncol, 2019, 9: 660) that researchers extracted cannflavin A and cannflavin C as well as other flavonoids from local Cannabis Sativa L. strain in Jamaica with chromatography. The researchers performed *in vitro* and *in vivo* experiments on the extracts successively. *In vivo* experiment results showed therapeutic effects of delaying local and metastatic tumor progression in pancreatic cancer animal models when cannflavin compounds were provided continuously by using smart radiotherapy biomaterials. Compared with the control group, repeated tests also showed a significant increase in survival of pancreatic cancer animals. Research results showed that cannflavin A and cannflavin C have important therapeutic potential for the treatment of pancreatic cancer, including therapeutic potential for radiosensitization and tumor metastasis. These results provide the basis for further study and optimization of the therapeutic results in clinical trials. The content of flavonoids in the cannabis sativa is much less than 1%, less than 0.14% on average, and the content of cannflavin A and cannflavin C in the cannabis sativa is extremely low. Therefore, it is infeasible to rely solely on cannabis sativa plant extraction to obtain these components. Currently, researchers are working on the preparation of these active factors based on biological systems, which will create a great deal of opportunities for cannflavins.

Barrett et al. reported in Cannflavin A and B, prenylated flavones from Cannabis sativa L. ([J]. Cellular&Molecular Life Sciences, 1986, 452-453) the separation of cannflavin A from an ethanol extract of cannabis sativa, but the yield of cannflavin A was extremely low.

Rea et al. reported in Biosynthesis of cannflavins A and B from Cannabis sativa L. ([J]. Phytochemistry, 2019, 134, 162-171) the synthesis of cannflavin A from dimethylallyl diphosphate under the catalysis of aromatic prenyltransferase of Sinorhizobium meliloti. However, this biosynthesis method is costly and not easily scalable industrially.

Patent document WO2017091837 discloses a method as follows: synthesizing a main chain of cannflavin compounds by using a known chemical synthesis technology with 2,4,6-trihydroxyacetophenone as a starting reactant, then forming a tricyclic structure by conjugating and ring closing, and finally performing a series of enzymatic modifications to obtain cannflavin A, cannflavin C and other flavonoids.

The chemical synthesis methods disclosed have the defects of long synthesis steps, multi-step reactions requiring silica gel column chromatography for purification, and difficulty in amplification. Therefore, there is an urgent need for a novel method for synthesizing cannflavin A and cannflavin C, which is simple, rapid, and easily scale-up.

### SUMMARY

Features of the present invention are set forth in the appended Claims.

The present invention provides a preparation method for a cannflavin compound. The preparation method has the advantages of cheap and easily available raw materials, few reaction steps, short production period, simple operation, etc.

The present invention aims to provide a preparation method for a cannflavin compound, which comprises the following steps (not part of the invention, only for illustration purposes): condensing 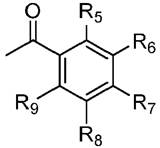 and diethyl carbonate under an alkaline condition to obtain 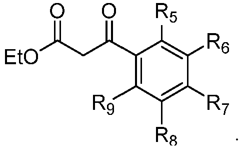 reacting 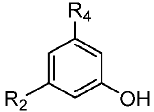 with an alcohol compound (R₃-OH) to obtain 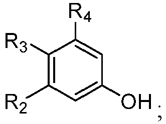 and reacting 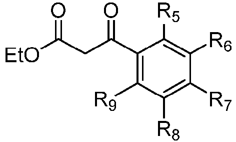 with 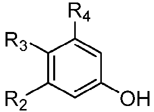 to obtain the cannflavin compound; wherein the cannflavin compound has a structure of general formula I:

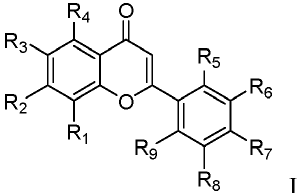

R₅, R₆, R₇, R₈, and R₉ are independently selected from the group consisting of: H, C₁₋₁₄ alkyl, C₃₋₁₄ alkenyl, hydroxy, C₁₋₅ alkoxy, carboxyl, amino, and halogen.

R₂ and R₄ are independently selected from the group consisting of: H, C₁₋₁₄ alkyl, C₃₋₁₄ alkenyl, hydroxy, C₁₋₅ alkoxy, carboxyl, and halogen.

R₃ and R₁ are independently selected from the group consisting of: H, C₁₋₁₄ alkyl, C₃₋₁₄ alkenyl, and C₁₋₅ alkoxy.

In one embodiment, R₃ is geranyl.

In one embodiment, R₁ is geranyl.

In one embodiment, R₂ is hydroxy.

In one embodiment, R₄ is hydroxy.

In one embodiment, R₇ is hydroxy.

In one embodiment, R₆ is methoxy.

In one embodiment, R₅ is H.

In one embodiment, R₈ is H.

In one embodiment, R₉ is H.

Furthermore, the preparation method comprises the following steps: condensing 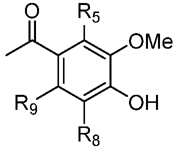 and diethyl carbonate under an alkaline condition to obtain 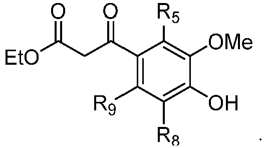 reacting 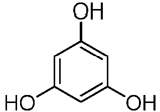 with an alcohol compound (R₃-OH) to obtain 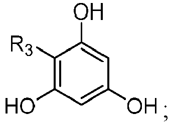 and reacting 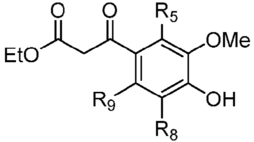 with 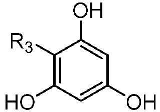 to obtain the cannflavin compound; wherein the cannflavin compound has a structure of general formula II: 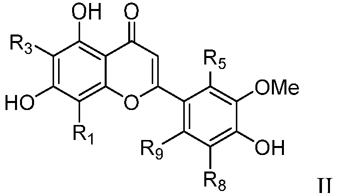
R₅, R₈, and R₉ are independently selected from the group consisting of: H, C₁₋₁₄ alkyl, C₃₋₁₄ alkenyl, hydroxy, C₁₋₅ alkoxy, carboxyl, amino, and halogen;
R₃ and R₁ are independently selected from the group consisting of: H, C₁₋₁₄ alkyl, C₃₋₁₄ alkenyl, and C₁₋₅ alkoxy.

Preferably, R₃ is geranyl; preferably, R₅ is H; preferably, R₈ is H; preferably, R₉ is H; and preferably, R₁ is geranyl.

The present invention also aims to provide a preparation method for cannflavin A and/or cannflavin C with 1,3,5-trihydroxybenzene and 4'-hydroxy-3'-methoxyacetophenone as starting materials, which comprises:
step (1): condensing 4'-hydroxy-3'-methoxyacetophenone and diethyl carbonate under an alkaline condition to obtain ethyl 4'-hydroxy-3'-methoxybenzoylacetate;
step (2): subjecting 1,3,5-trihydroxybenzene and geraniol to a C-alkylation reaction to obtain (*E*)-2-(3,7-dimethyloct-2,6-dien-1-yl)benzene-1,3,5-triphenol; and
step (3): condensing ethyl 4'-hydroxy-3'-methoxybenzoylacetate and (*E*)-2-(3,7-dimethyloct-2,6-dien-1-yl)benzene-1,3,5-triphenol at high temperature to produce cannflavin A and/or cannflavin C.

The reaction equation of the step (2) is as follows:

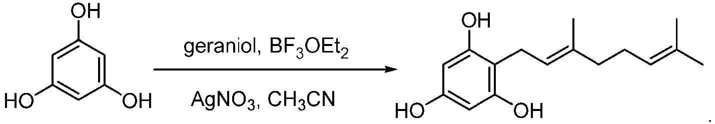

Preferably, in the step (2), the reaction is performed under an oxygen-free environment, and further, the inert gas is nitrogen or argon; the reaction system further comprises a solvent which is acetonitrile and/or diethyl ether; and in one embodiment of the present invention, the solvent is acetonitrile.

Preferably, in the step (2), the molar ratio of 1,3,5-trihydroxybenzene to geraniol is 1:3 to 3:1, and further, the molar ratio of 1,3,5-trihydroxybenzene to geraniol is 1:3, 1:2, 1:1, 2:1 or 3:1, and most preferably, 1:1.

Preferably, in the step (2), the reaction temperature is 0-25°C, and further, the reaction temperature is 0°C, 5°C, 10°C, 15°C, 20°C, or 25°C.

Preferably, in the step (2), the reaction time is 8-24 h, and further, the reaction time is 8 h, 12 h, 16 h, 20 h, or 24 h, and most preferably, 12 h.

Preferably, in the step (2), the reaction further requires the dropwise addition of a solution of boron trifluoride in diethyl ether.

In one embodiment of the present invention, the step (2) comprises the following specific steps: adding acetonitrile saturated with silver nitrate into a reaction vessel, then adding 1,3,5-trihydroxybenzene and geraniol, cooling under inert gas protection, adding dropwise a solution of boron trifluoride in diethyl ether, stirring, reacting, and then performing post-treatment on the resulting reaction liquid to obtain (*E*)-2-(3,7-dimethyloct-2,6-dien-1-yl)benzene-1,3,5-triphenol.

Preferably, the post-treatment comprises a quenching step.

More preferably, the quenching comprises the following specific step: adding an ice-water mixture to the reaction vessel.

More preferably, the quenching solvent is one or more selected from the group consisting of: an ice-water mixture, methyl tert-butyl ether, diethyl ether, ethyl acetate, and butyl acetate; and in one embodiment of the present invention, the quenching solvent is an ice-water mixture.

Preferably, the post-treatment further comprises an extraction step after the quenching step.

More preferably, the extraction solvent is one or more selected from the group consisting of: methyl *tert*-butyl ether, diethyl ether, ethyl acetate, and butyl acetate; and in one embodiment of the present invention, the extraction solvent is ethyl acetate.

More preferably, the extraction temperature is 5-40°C, and further preferably 15-25°C, and further, the extraction temperature is 15°C, 20°C, or 25°C.

In one embodiment of the present invention, the extraction step comprises the following specific steps: adding an extraction solvent into the reaction liquid, and layering; adding an extraction solvent to the aqueous layer for extraction, and layering; and combining the organic layers, washing, layering, and then combining the organic layers.

Preferably, the post-treatment further comprises a distillation step after the extraction step; and more preferably, the distillation step comprises steps of atmospheric distillation and vacuum distillation. Preferably, the vacuum distillation temperature is 20-50°C, and the vacuum degree is -0.1 MPa to -0.05 MPa.

Preferably, the post-treatment further comprises a column chromatography step in sequence after the distillation step; and more preferably, the column chromatography step comprises normal-phase column chromatography and reverse-phase column chromatography.

More preferably, the column chromatography is normal-phase column chromatography, and the eluent in the column chromatography is selected from the group consisting of: petroleum ether, n-hexane, ethyl acetate, toluene, triethylamine, dichloromethane, water, and methanol; and in one embodiment of the present invention, the eluent is petroleum ether (PE) and ethyl acetate (EA), and the ratio of the eluent is PE:EA = 8:1→4:1→2:1.

In a preferred embodiment of the present invention, the post-treatment comprises: quenching, extraction, distillation, and column chromatography in sequence, wherein the specific operation and conditions of each step are as described above in the present invention.

In one embodiment of the present invention, the preparation method for (*E*)-2-(3,7-dimethyloct-2,6-dien-1-yl)benzene-1,3,5-triphenol comprises the following steps:
(2-1) reaction step: adding acetonitrile saturated with silver nitrate into a reaction vessel, then adding 1,3,5-trihydroxybenzene and geraniol, cooling under inert gas protection, adding dropwise a solution of boron trifluoride in diethyl ether, stirring, heating to room temperature, and reacting for 8-24 h;
(2-2) control step: adding an ice-water mixture into the mixture of (2-1), and layering; adding ethyl acetate to the aqueous layer for extraction, and layering; combining the organic layers, washing with saturated brine, and layering; and combining the organic layers, drying over anhydrous sodium sulfate, filtering under vacuum, and concentrating the filtrate; and
(2-3): separating the concentrate of the step (2-2) by column chromatography (silica gel 200-300 mesh, PE:EA = 8:1→4:1→2:1) to obtain (*E*)-2-(3,7-dimethyloct-2,6-dien-1-yl)benzene-1,3,5-triphenol.

The reaction equation of the step (3) is as follows:

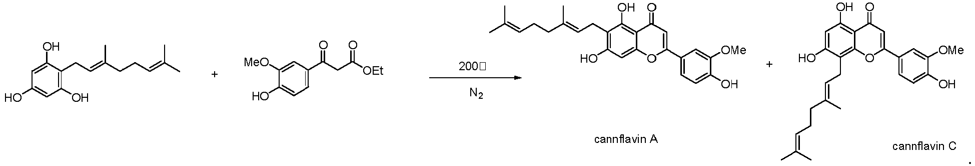

Preferably, in the step (3), the reaction environment is an oxygen-free environment; more preferably, the reaction environment is an inert gas protected reaction environment; and in one embodiment of the present invention, the reaction environment is a nitrogen protected reaction environment.

Preferably, in the step (3), the reaction temperature is 150-230°C; further, the reaction temperature is 160°C, 180°C, 200°C, or 220°C; and in one embodiment of the present invention, the reaction temperature is 200°C.

Preferably, in the step (3), the reaction time is 2-8 h; further, the reaction time is 3 h, 5 h, or 7 h; and in one embodiment of the present invention, the reaction time is 3 h.

Preferably, in the step (3), the molar ratio of ethyl 4'-hydroxy-3'-methoxybenzoylacetate to (*E*)-2-(3,7-dimethyloct-2,6-dien-1-yl)benzene-1,3,5-triphenol is 1:3 to 3:1, and further, the molar ratio of ethyl 4'-hydroxy-3'-methoxybenzoylacetate to (*E*)-2-(3,7-dimethyloct-2,6-dien-1-yl)benzene-1,3,5-triphenol is 1:3, 1:2, 1:1, 1.5:1, 2:1, or 3:1, and most preferably, 1.5:1.

In one embodiment of the present invention, the step (3) comprises the following specific steps: under inert gas protection, adding ethyl 4'-hydroxy-3'-methoxybenzoylacetate and (*E*)-2-(3,7-dimethyloct-2,6-dien-1-yl)benzene-1,3,5-triphenol into a reaction vessel, stirring, heating, reacting, and then performing post-treatment on the resulting reaction liquid to obtain cannflavin A and/or cannflavin C.

Preferably, the post-treatment further comprises a column chromatography step; more preferably, the column chromatography step comprises normal-phase column chromatography and reverse-phase column chromatography.

More preferably, the column chromatography step comprises the following steps: subjecting the mixture to normal-phase silica gel column chromatography multiple times, followed by reverse-phase silica gel column chromatography multiple times.

More preferably, the eluent in the column chromatography is selected from the group consisting of: petroleum ether, *n*-hexane, ethyl acetate, toluene, triethylamine, dichloromethane, water, and methanol; in one embodiment of the present invention, the eluent in the normal-phase silica gel column chromatography is petroleum ether (PE) and ethyl acetate (EA), and the ratio of the eluent is PE:EA = 10:1→2:1, and the eluent in the reverse-phase silica gel column chromatography is water and methanol, and the ratio of the eluent is water: methanol = 50:50→10:90.

Preferably, the post-treatment further comprises a recrystallization step after the column chromatography step; and more preferably, the recrystallization step comprises steps of dissolution and cooling to precipitate.

More preferably, the solvent for dissolution is one or a mixture of more selected from the group consisting of: toluene, methanol, acetone, water, etc.; and in one embodiment of the present invention, the solvent is methanol.

More preferably, the dissolution temperature is 40-100°C, and further preferably, 60-100°C; and in one embodiment of the present invention, the dissolution temperature is 65°C.

More preferably, the step of cooling to precipitate comprises cooling to 1-30°C; and in one embodiment of the present invention, the step of cooling to precipitate comprises cooling to 18-22°C.

In one embodiment of the present invention, the recrystallization step comprises the following steps: adding a solvent, heating to 40-100°C, dissolving the solid completely, cooling to 1-30°C, precipitating, and filtering the precipitate.

Preferably, the post-treatment further comprises a drying step after the recrystallization step.

More preferably, the drying step is vacuum drying; and more preferably, the vacuum degree is -0.08 MPa to -0.01 MPa.

More preferably, the drying temperature is 45-65°C.

In a preferred embodiment of the present invention, the post-treatment comprises: column chromatography, recrystallization, and drying in sequence, wherein the specific operation and conditions of each step are as described above in the present invention.

In one specific embodiment of the present invention, the preparation method for cannflavin A and/or cannflavin C comprises the following steps:
(3-1) reaction step: under nitrogen protection, adding ethyl 4'-hydroxy-3'-methoxybenzoylacetate and (*E*)-2-(3,7-dimethyloct-2,6-dien-1-yl)benzene-1,3,5-triphenol into a reaction vessel, stirring, heating to 150-230°C, and reacting for 2-8 h;
(3-2): cooling to room temperature, subjecting the mixture to normal-phase silica gel column chromatography multiple times, followed by reverse-phase silica gel column chromatography multiple times, and evaporating the product to dryness;
(3-3): adding methanol into the evaporated product obtained in the step (3-2), heating to 65°C, dissolving the solid completely, cooling to 18-22°C, precipitating the solid, filtering, and rinsing the filter cake; and
(3-4): drying the filter cake obtained in the step (3-3) under vacuum at 50-55°C.

Preferably, ethyl 4'-hydroxy-3'-methoxybenzoylacetate is prepared by the following step: condensing 4'-hydroxy-3'-methoxyacetophenone and diethyl carbonate under an alkaline condition to obtain ethyl 4'-hydroxy-3'-methoxybenzoylacetate.

The reaction equation of the step (1) is as follows:

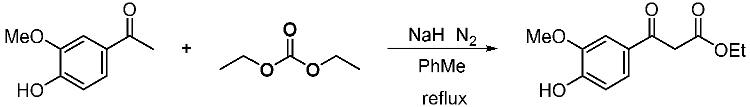

Preferably, in the step (1), the reaction is performed under an alkaline condition, and more preferably, the alkaline condition is that an alkali, such as an inorganic alkali, is included in the reaction system, and the inorganic alkali is one or a mixture of more selected from the group consisting of: pure inorganic alkali of sodium hydride and paraffin oil-coated sodium hydride; and in one embodiment of the present invention, the inorganic alkali is paraffin oil-coated sodium hydride.

More preferably, the mass molar ratio of the alkali to 4'-hydroxy-3'-methoxyacetophenone is 50-1000 g/mol, and further preferably, 100-150 g/mol; and further, the mass molar ratio of the alkali to 4'-hydroxy-3'-methoxyacetophenone is 100 g/mol, 110 g/mol, 120 g/mol, 130 g/mol, 140 g/mol, or 150 g/mol.

Preferably, in the step (1), the reaction system further comprises a solvent selected from the group consisting of: toluene and/or benzene: and in one embodiment of the present invention, the solvent is toluene.

Preferably, in the step (1), the molar ratio of 4'-hydroxy-3'-methoxyacetophenone to diethyl carbonate is 1:3 to 3:1, and further, the molar ratio of 4'-hydroxy-3'-methoxyacetophenone to diethyl carbonate is 1:3, 1:2, 1:1, 2:1, or 3:1, and most preferably, 1:2.

Preferably, in the step (1), the reaction environment is an oxygen-free environment; more preferably, the reaction environment is an inert gas protected reaction environment; and in one embodiment of the present invention, the reaction environment is a nitrogen protected reaction environment.

Preferably, in the step (1), the reaction temperature is 80-130°C, and further, the reaction temperature is 80°C, 90°C, 100°C, 110°C, 120°C, or 130°C.

Preferably, in the step (1), the reaction time is 2-12 h, and further, the reaction time is 4 h, 6 h, 8 h, 10 h, or 12 h, and most preferably, 4 h.

Preferably, in the step (1), 4'-hydroxy-3'-methoxyacetophenone is added dropwise to diethyl carbonate.

In one embodiment of the present invention, the preparation method comprises the following specific steps: under nitrogen protection, adding 60% NaH and toluene into a reaction vessel, stirring, adding diethyl carbonate, heating, adding dropwise a mixture of 4'-hydroxy-3'-methoxyacetophenone and toluene, stirring, reacting, and then performing post-treatment on the resulting reaction liquid to obtain ethyl 4'-hydroxy-3'-methoxybenzoylacetate.

Preferably, the post-treatment comprises a quenching step.

More preferably, the quenching comprises the following specific step: adding an acid to the reaction vessel to adjust pH of the mixture to 4.0-7.0.

More preferably, the acid is an inorganic acid which is one or a mixture of more selected from the group consisting of: pure inorganic acid of acetic acid and an aqueous solution thereof; and in one embodiment of the present invention, the acid is acetic acid.

Preferably, the post-treatment further comprises an extraction step after the quenching step.

More preferably, the extraction solvent is one or more selected from the group consisting of: methyl tert-butyl ether, diethyl ether, ethyl acetate, and butyl acetate; and in one embodiment of the present invention, the extraction solvent is ethyl acetate.

More preferably, an extraction temperature is 5-40°C, and further preferably, 15-25°C.

In one embodiment of the present invention, the extraction step comprises the following specific steps: adding an extraction solvent into the reaction liquid, and layering; adding an extraction solvent to the aqueous layer for extraction, and layering; and combining the organic layers, washing, layering, and then combining the organic layers.

Preferably, the post-treatment further comprises a distillation step after the extraction step; and more preferably, the distillation step comprises steps of atmospheric distillation and vacuum distillation. Preferably, the vacuum distillation temperature is 20-50°C, and the vacuum degree is -0.1 MPa to -0.05 MPa.

Preferably, the post-treatment further comprises washing in sequence after the distillation step; more preferably, the washing solvent is selected from the group consisting of: n-hexane and/or diethyl ether; and in one embodiment of the present invention, the washing solvent is n-hexane.

In a preferred embodiment of the present invention, the post-treatment comprises: quenching, extraction, distillation and washing in sequence, wherein the specific operation and conditions of each step are as described above in the present invention.

In one embodiment of the present invention, the preparation method for ethyl 4'-hydroxy-3'-methoxybenzoylacetate comprises the following steps:
(1-1) reaction step: under nitrogen protection, adding 60% NaH and toluene into a reaction vessel, stirring, adding diethyl carbonate, heating to 80-130°C, adding dropwise a mixture of 4'-hydroxy-3'-methoxyacetophenone and toluene, stirring, and reacting for 2-12 h;
(1-2) control step: adding acetic acid to the mixture of (1-1) to adjust pH to 4.0-7.0, adding ethyl acetate, and layering; adding ethyl acetate to the aqueous layer for extraction, layering, combining the organic layers, washing with saturated ammonium chloride aqueous solution and saturated brine, layering, combining the organic layers, drying over anhydrous sodium sulfate, filtering under vacuum, and concentrating the filtrate; and
(1-3) adding the concentrate obtained in the step (1-2) into n-hexane, stirring for 2-12 h, and filtering to obtain ethyl 4'-hydroxy-3'-methoxybenzoylacetate.

The present invention further provides a preparation method for cannflavin compounds, which comprises the preparation step of (*E*)-2-(3,7-dimethyloct-2,6-dien-1-yl)benzene-1,3,5-triphenol described above.

The present invention further provides use of ethyl 4'-hydroxy-3'-methoxybenzoylacetate in preparing a compound, wherein the compound is cannflavin A and/or cannflavin C.

The present invention has the advantages of cheap and easily available raw materials, simple synthetic route, simple operation, short production period, and relatively low synthetic cost, and thus has certain economic value.

### DETAILED DESCRIPTION

It should be noted that the detailed description as follows is exemplary and is intended to provide further explanation of the present application. Unless otherwise stated, all technical and scientific terms used herein have the same meaning as that commonly understood by those of ordinary skill in the art to which the present application belongs.

### Example 1: Synthesis of Ethyl 4'-hydroxy-3'-methoxybenzoylacetate

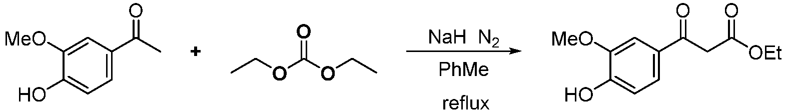

To a reaction flask, 60% NaH (6.00 g, 150 mmol) was added, under nitrogen protection, toluene (60 mL) and DEC (11.82 g, 100 mmol) were added, and the resulting mixture was stirred, and heated to reflux. A solution of 4'-hydroxy-3'-methoxyacetophenone (12.82 g, 50 mmol) in toluene (60 mL) was added dropwise. After the addition was completed, the resulting mixture was refluxed for 4 h, then cooled to room temperature, adjusted to pH neutral with acetic acid, and added with a saturated ammonium chloride aqueous solution (150 mL). The aqueous phase was extracted with ethyl acetate (100 mL × 3). The organic phases were combined, and washed successively with a saturated ammonium chloride aqueous solution (100 mL × 2) and saturated brine (100 mL × 2). The resulting organic phase was dried over anhydrous sodium sulfate, and filtered under vacuum. The filtrate was concentrated to dryness, added with *n*-hexane (50 mL), stirred overnight, and filtered to obtain ethyl 4'-hydroxy-3'-methoxybenzoylacetate (15.43 g, 93.9%) as an orange-yellow solid. MS (ESI): 238.9 [M+H]⁺.

### Example 2: Synthesis of (E)-2-(3,7-dimethyloct-2,6-dien-1-yl)benzene-1,3,5-triphenol

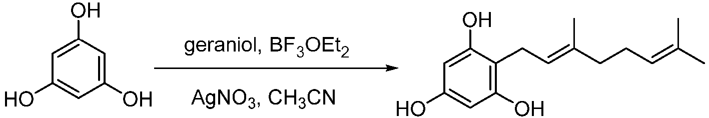

To a reaction flask, 50 mL of acetonitrile saturated with silver nitrate was added and stirred. 1,3,5-trihydroxybenzene (3.33 g, 26.41 mmol) and geraniol (4.08 g, 26.45 mmol) were added. Under nitrogen protection, the resulting mixture was cooled in an ice-water bath, followed by dropwise addition of a solution of boron trifluoride in diethyl ether (1.23 g, 8.85 mmol). After the addition was completed, the resulting mixture was stirred at room temperature overnight. The reaction liquid was poured into 50 mL of ice-water mixture, and extracted with ethyl acetate (30 mL × 3). The organic phases were combined, washed successively with 5% aqueous sodium bicarbonate solution and water (20 mL × 3), dried over anhydrous sodium sulfate, and filtered under vacuum. The filtrate was concentrated to dryness, and the resulting oil was subjected to column chromatography (silica gel 200-300 mesh, petroleum ether:ethyl acetate = 8:1-4:1-2:1) to obtain (*E*)-2-(3,7-dimethyloct-2,6-dien-1-yl)benzene-1,3,5-triphenol as an oil (0.6 g). MS (ESI): 263.2 [M+H]⁺.

### Example 3: Synthesis of Cannflavin A and/or Cannflavin C

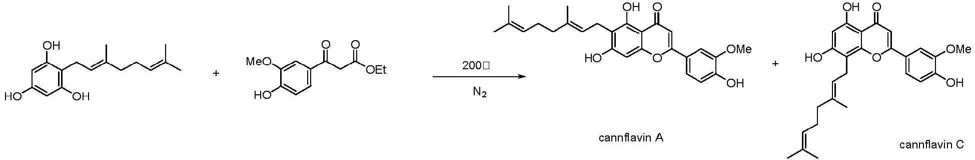

To a reaction flask, (*E*)-2-(3,7-dimethyloct-2,6-dien-1-yl)benzene-1,3,5-triphenol (3.8 g, 15.95 mmol) and ethyl 4'-hydroxy-3'-methoxybenzoylacetate (2.8 g, 10.67 mmol) were added and stirred. Under nitrogen protection, the resulting mixture was heated to 200°C, and maintained at this temperature for 3 h. The reaction mixture was cooled to room temperature, and the resulting product was purified by normal-phase silica gel column chromatography (silica gel 200-300 mesh, petroleum ether:ethyl acetate = 10:1-2:1) multiple times and then purified by reverse-phase silica gel column chromatography (reverse-phase silica gel 200-300 mesh, water:methanol = 50:50→10:90) multiple times to obtain cannflavin A (0.3227 g) and cannflavin C (0.2048 g) as yellow solids.

Cannflavin A: MS (ESI): MS (ESI): 437.0[M+H]⁺, ¹H-NMR (Acetone-d₆, 400 MHz), δ:1.56 (3H, s), 1.61 (3H, s), 1.81 (3H, s), 1.96 (2H, m), 2.04 (2H, m), 3.37 (2H, d), 4.00 (3H, s), 5.06 (1H, t), 5.29 (1H, t), 6.62 (1H, s), 6.70 (1H, s), 7.00 (1H, d), 7.58 (1H, dd), 7.60 (1H, d), 8.49 (1H, s), 9.56 (1H, s), 13.05. (1H, s).

Cannflavin C: MS (ESI): 437.0[M+H]⁺, ¹H-NMR (Acetone-d₆, 400 MHz), δ:1.50 (3H, s), 1.52 (3H, s), 1.84 (3H, s), 1.97 (2H, m), 2.03 (2H, m), 359(2H, d), 4.01 (3H, s), 5.04 (1H, t), 5.36 (1H, t), 6.35 (1H, s), 6.72 (1H, s), 7.03 (1H, d), 7.63 (1H, dd), 7.64(1H, d), 8.54 (1H, s), 9.63 (1H, s), 12.98. (1H, s).

## Claims

1. A preparation method for a cannflavin compound, comprising the following steps:
step (1): condensing 4'-hydroxy-3'-methoxyacetophenone and diethyl carbonate under an alkaline condition to obtain ethyl 4'-hydroxy-3'-methoxybenzoylacetate;
step (2): subjecting 1,3,5-trihydroxybenzene and geraniol to a C-alkylation reaction to obtain (E)-2-(3,7-dimethyloct-2,6-dien-1-yl)benzene-1,3,5-triphenol; and
step (3): condensing ethyl 4'-hydroxy-3'-methoxybenzoylacetate and (E)-2-(3,7-dimethyloct-2,6-dien-1-yl)benzene-1,3,5-triphenol at high temperature to produce cannflavin A and/or cannflavin C;
the reaction temperature in the step (3) is 180-230°C;
cannflavin A represented by a formula: 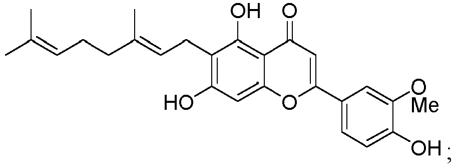
cannflavin C represented by a formula:

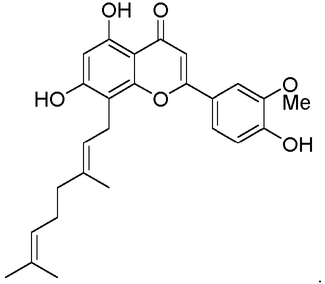

2. The preparation method according to claim 1, wherein the step (1) comprises a step of reacting 4'-hydroxy-3'-methoxyacetophenone with diethyl carbonate; the reaction is performed under an oxygen-free environment; the reaction is performed under an alkaline condition; the reaction system further comprises a solvent selected from the group consisting of: toluene and benzene.

3. The preparation method according to claim 2, wherein the oxygen-free environment in the step (1) is an inert gas protected reaction environment;
the alkali is an inorganic alkali;
a mass molar ratio of the inorganic alkali to 4'-hydroxy-3'-methoxyacetophenone is 50-1000 g/mol;
a molar ratio of 4'-hydroxy-3'-methoxyacetophenone to diethyl carbonate is 1:3 to 3:1;
the reaction temperature is 80-130°C;
the reaction time is 2-12 h; and
4'-hydroxy-3'-methoxyacetophenone is added dropwise to diethyl carbonate.

4. The preparation method according to claim 1, wherein the step (1) comprises the following steps:
under inert gas protection, adding NaH and toluene into a reaction vessel, stirring, adding diethyl carbonate, heating, adding dropwise a mixture of 4'-hydroxy-3'-methoxyacetophenone and toluene, stirring, reacting, and then performing post-treatment on the resulting reaction liquid to obtain ethyl 4'-hydroxy-3'-methoxybenzoylacetate;
the post-treatment comprises a quenching step;
the quenching step comprises: adding an acid to the reaction vessel to adjust pH of the mixture to 4.0-7.0;
the acid is an inorganic acid, and the acid is one or a mixture of more selected from the group consisting of: pure inorganic acid of acetic acid and an aqueous solution thereof.

5. The preparation method according to claim 1, wherein the step (2) comprises a step of subjecting 1,3,5-trihydroxybenzene and geraniol to the C-alkylation reaction, wherein the reaction is performed under an oxygen-free environment; and the reaction system further comprises a solvent selected from the group consisting of: acetonitrile and diethyl ether.

6. The preparation method according to claim 5, wherein the oxygen-free environment in the step (2) is an inert gas protected reaction environment, the inert gas is nitrogen;
a molar ratio of 1,3,5-trihydroxybenzene to geraniol is 1:3 to 3:1;
the reaction temperature is 0-25°C;
the reaction time is 8-24 h; and
the reaction further requires dropwise addition of a solution of boron trifluoride in diethyl ether.

7. The preparation method according to claim 1, wherein the step (2) comprises the following steps:
adding acetonitrile saturated with silver nitrate into a reaction vessel, then adding 1,3,5-trihydroxybenzene and geraniol, cooling under inert gas protection, adding dropwise a solution of boron trifluoride in diethyl ether, stirring, reacting, and then performing post-treatment on the resulting reaction liquid to obtain (*E*)-2-(3,7-dimethyloct-2,6-dien-1-yl)benzene-1,3,5-triphenol;
the post-treatment comprises a quenching step;
the quenching step comprises: adding a quenching solvent to the reaction vessel;
wherein the quenching solvent is one or more selected from the group consisting of: an ice-water mixture, methyl *tert*-butyl ether, diethyl ether, ethyl acetate, and butyl acetate.

8. The preparation method according to claim 1, wherein the step (3) comprises a step of reacting ethyl 4'-hydroxy-3'-methoxybenzoylacetate with (*E*)-2-(3,7-dimethyloct-2,6-dien-1-yl)benzene-1,3,5-triphenol, wherein the reaction is performed under an oxygen-free environment.

9. The preparation method according to claim 8, wherein the oxygen-free environment in the step (3) is an inert gas protected reaction environment;
the reaction time is 2-8 h; and
a molar ratio of ethyl 4'-hydroxy-3'-methoxybenzoylacetate to (*E*)-2-(3,7-dimethyloct-2,6-dien-1-yl)benzene-1,3,5-triphenol is 1:3 to 3:1.

10. The preparation method according to claim 1, wherein the step (3) comprises the following steps: under inert gas protection, adding ethyl 4'-hydroxy-3'-methoxybenzoylacetate and (*E*)-2-(3,7-dimethyloct-2,6-dien-1-yl)benzene-1,3,5-triphenol into a reaction vessel, stirring, heating, reacting, and then performing post-treatment on the resulting reaction liquid to obtain cannflavin A and/or cannflavin C.

11. The preparation method according to claim 1, comprising the following steps:
condensing 4'-hydroxy-3'-methoxyacetophenone and diethyl carbonate under an alkaline condition to obtain ethyl 4'-hydroxy-3'-methoxybenzoylacetate;
adding acetonitrile saturated with silver nitrate into a reaction vessel, then adding 1,3,5-trihydroxybenzene and geraniol, cooling under inert gas protection, adding dropwise a solution of boron trifluoride in diethyl ether, stirring, reacting, and then performing post-treatment on the resulting reaction liquid to obtain (*E*)-2-(3,7-dimethyloct-2,6-dien-1-yl)benzene-1,3,5-triphenol;
under inert gas protection, adding ethyl 4'-hydroxy-3'-methoxybenzoylacetate and (*E*)-2-(3,7-dimethyloct-2,6-dien-1-yl)benzene-1,3,5-triphenol into a reaction vessel, stirring, heating, reacting,
and then performing post-treatment on the resulting reaction liquid to obtain cannflavin A and/or cannflavin C.

12. Use of ethyl 4'-hydroxy-3'-methoxybenzoylacetate in preparing a compound, wherein the compound is cannflavin A and/or cannflavin C;
wherein ethyl 4'-hydroxy-3'-methoxybenzoylacetate is reacted with (*E*)-2-(3,7-dimethyloct-2,6-dien-1-yl)benzene-1,3,5-triphenol at high temperature;
the reaction temperature is 180-230°C;
cannflavin A represented by a formula: 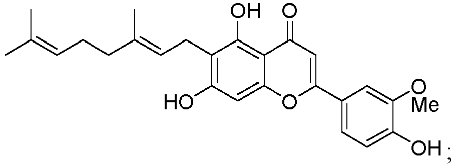
cannflavin C represented by a formula:

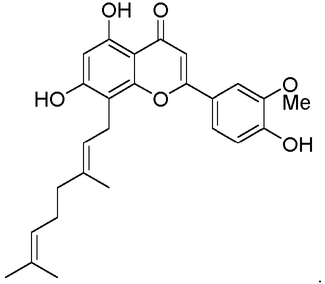

## Patentansprüche

1. Verfahren zur Herstellung einer Cannflavinverbindung, umfassend die folgenden Schritte:
Schritt (1): Kondensierenlassen von 4'-Hydroxy-3'-methoxyacetophenon und Diethylcarbonat unter einer alkalischen Bedingung, um Ethyl-4 '-hydroxy-3'-methoxybenzoylacetat zu erhalten;
Schritt (2): Aussetzen von 1,3,5-Trihydroxybenzol und Geraniol gegenüber einer C-Alkylierungsreaktion, um (*E*)-2-(3,7-Dimethyloct-2,6-dien-1-yl)benzol-1,3,5-triphenol zu erhalten; und
Schritt (3): Kondensierenlassen von Ethyl-4'-hydroxy-3'-methoxybenzoylacetat und (*E*)-2-(3,7-Dimethyloct-2,6-dien-1-yl)benzol-1,3,5-triphenol bei hoher Temperatur, um Cannflavin A und/oder Cannflavin C zu produzieren;
die Reaktionstemperatur in dem Schritt (3) beträgt 180-230 °C;
Cannflavin A, dargestellt durch eine Formel: 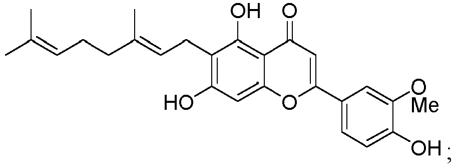
Cannflavin C, dargestellt durch eine Formel:

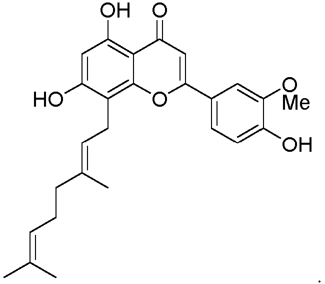

2. Verfahren zur Herstellung nach Anspruch 1, wobei der Schritt (1) einen Schritt des Reagierenlassens von 4'-Hydroxy-3'-methoxyacetophenon mit Diethylcarbonat umfasst; die Reaktion unter einer sauerstofffreien Umgebung durchgeführt wird; die Reaktion unter einer alkalischen Bedingung durchgeführt wird; das Reaktionssystem ferner ein Lösungsmittel umfasst, das aus der Gruppe ausgewählt ist, die aus Folgendem besteht: Toluol und Benzol.

3. Verfahren zur Herstellung nach Anspruch 2, wobei die sauerstofffreie Umgebung in dem Schritt (1) eine durch Inertgas geschützte Reaktionsumgebung ist;
das Alkali ein anorganisches Alkali ist;
ein Molmassenverhältnis des anorganischen Alkalis zu 4'-Hydroxy-3'-methoxyacetophenon 50-1000 g/mol beträgt;
ein Molverhältnis von 4'-Hydroxy-3'-methoxyacetophenon zu Diethylcarbonat 1:3 bis 3:1 beträgt; die Reaktionstemperatur 80-130 °C beträgt;
die Reaktionszeit 2-12 h beträgt; und
4'-Hydroxy-3'-methoxyacetophenon tropfenweise zu Diethylcarbonat hinzugefügt wird.

4. Verfahren zur Herstellung nach Anspruch 1, wobei der Schritt (1) die folgenden Schritte umfasst: unter Inertgasschutz, Hinzufügen von NaH und Toluol in ein Reaktionsgefäß, Rühren, Hinzufügen von Diethylcarbonat, Erhitzen, tropfenweises Hinzufügen einer Mischung aus 4'-Hydroxy-3'-methoxyacetophenon und Toluol, Rühren, Reagierenlassen und dann Durchführen einer Nachbehandlung an der resultierenden Reaktionsflüssigkeit, um Ethyl-4'-hydroxy-3'-methoxybenzoylacetat zu erhalten;
die Nachbehandlung umfasst einen Abschreckschritt;
der Abschreckschritt umfasst Folgendes: Hinzufügen einer Säure zu dem Reaktionsgefäß, um den pH-Wert der Mischung auf 4,0-7,0 einzustellen;
die Säure ist eine anorganische Säure und bei der Säure handelt es sich um eine oder eine Mischung aus mehreren, die aus der Gruppe ausgewählt sind, die aus Folgendem besteht: reiner anorganischer Säure der Essigsäure und einer wässrigen Lösung davon.

5. Verfahren zur Herstellung nach Anspruch 1, wobei der Schritt (2) einen Schritt des Aussetzens des 1,3,5-Trihydroxybenzols und Geraniols gegenüber der C-Alkylierungsreaktion umfasst, wobei die Reaktion unter einer sauerstofffreien Umgebung durchgeführt wird; und das Reaktionssystem ferner ein Lösungsmittel umfasst, das aus der Gruppe ausgewählt ist, die aus Folgendem besteht: Acetonitril und Diethylether.

6. Verfahren zur Herstellung nach Anspruch 5, wobei die sauerstofffreie Umgebung in dem Schritt (2) eine durch Inertgas geschützte Reaktionsumgebung ist, das Inertgas Stickstoff ist;
ein Molverhältnis von 1,3,5-Trihydroxybenzol zu Geraniol 1:3 bis 3:1 beträgt;
die Reaktionstemperatur 0-25 °C beträgt;
die Reaktionszeit 8-24 h beträgt; und
die Reaktion ferner ein tropfenweises Hinzufügen einer Lösung von Bortrifluorid in Diethylether erforderlich macht.

7. Verfahren zur Herstellung nach Anspruch 1, wobei der Schritt (2) die folgenden Schritte umfasst: Hinzufügen von mit Silbernitrat gesättigtem Acetonitril in ein Reaktionsgefäß, dann Hinzufügen von 1,3,5-Trihydroxybenzol und Geraniol, Abkühlenlassen unter Inertgasschutz, tropfenweises Hinzufügen einer Lösung von Bortrifluorid in Diethylether, Rühren, Reagierenlassen und dann Durchführen einer Nachbehandlung an der resultierenden Reaktionsflüssigkeit, um (*E*)-2-(3,7-Dimethyloct-2,6-dien-1-yl)benzol-1,3,5-triphenol zu erhalten;
die Nachbehandlung umfasst einen Abschreckschritt;
der Abschreckschritt umfasst Folgendes: Hinzufügen eines Abschrecklösungsmittels zu dem Reaktionsgefäß;
wobei es sich bei dem Abschrecklösungsmittel um eines oder mehrere handelt, die aus der Gruppe ausgewählt sind, die aus Folgendem besteht: einer Eis-Wasser-Mischung, Methyl-*tert*-butylether, Diethylether, Ethylacetat und Butylacetat.

8. Verfahren zur Herstellung nach Anspruch 1, wobei der Schritt (3) einen Schritt des Reagierenlassens von Ethyl-4'-hydroxy-3'-methoxybenzoylacetat mit (*E*)-2-(3,7-dimethyloct-2,6-dien-1-yl)benzol-1,3,5-triphenol umfasst, wobei die Reaktion unter einer sauerstofffreien Umgebung durchgeführt wird.

9. Verfahren zur Herstellung nach Anspruch 8, wobei die sauerstofffreie Umgebung in dem Schritt (3) eine durch Inertgas geschützte Reaktionsumgebung ist;
die Reaktionszeit 2-8 h beträgt; und
ein Molverhältnis von Ethyl-4'-hydroxy-3'-methoxybenzoylacetat zu (*E*)-2-(3,7-dimethyloct-2,6-dien-1-yl)benzol-1,3,5-triphenol 1:3 bis 3:1 beträgt.

10. Verfahren zur Herstellung nach Anspruch 1, wobei der Schritt (3) die folgenden Schritte umfasst: unter Inertgasschutz, Hinzufügen von Ethyl-4'-hydroxy-3'-methoxybenzoylacetat und (*E*)-2-(3,7-dimethyloct-2,6-dien-1-yl)benzol-1,3,5-triphenol in ein Reaktionsgefäß, Rühren, Erhitzen, Reagierenlassen und dann Durchführen einer Nachbehandlung an der resultierenden Reaktionsflüssigkeit, um Cannflavin A und/oder Cannflavin C zu erhalten.

11. Verfahren zur Herstellung nach Anspruch 1, umfassend die folgenden Schritte:
Kondensierenlassen von 4'-Hydroxy-3'-methoxyacetophenon und Diethylcarbonat unter einer alkalischen Bedingung, um Ethyl-4'-hydroxy-3'-methoxybenzoylacetat zu erhalten;
Hinzufügen von mit Silbernitrat gesättigtem Acetonitril in ein Reaktionsgefäß, dann Hinzufügen von 1,3,5-Trihydroxybenzol und Geraniol, Abkühlenlassen unter Inertgasschutz, tropfenweises Hinzufügen einer Lösung von Bortrifluorid in Diethylether, Rühren, Reagierenlassen und dann Durchführen einer Nachbehandlung an der resultierenden Reaktionsflüssigkeit, um (*E*)-2-(3,7-Dimethyloct-2,6-dien-1-yl)benzol-1,3,5-triphenol zu erhalten;
unter Inertgasschutz, Hinzufügen von Ethyl-4'-hydroxy-3'-methoxybenzoylacetat und (*E*)-2-(3,7-dimethyloct-2,6-dien-1-yl)benzol-1,3,5-triphenol in ein Reaktionsgefäß, Rühren, Erhitzen, Reagierenlassen und dann Durchführen einer Nachbehandlung an der resultierenden Reaktionsflüssigkeit, um Cannflavin A und/oder Cannflavin C zu erhalten.

12. Verwendung von Ethyl-4'-hydroxy-3'-methoxybenzoylacetat bei der Herstellung einer Verbindung, wobei die Verbindung Cannflavin A und/oder Cannflavin C ist;
wobei Ethyl-4'-hydroxy-3'-methoxybenzoylacetat mit (*E*)-2-(3,7-dimethyloct-2,6-dien-1-yl)benzol-1,3,5-triphenol bei hoher Temperatur reagieren gelassen wird;
die Reaktionstemperatur 180-230 °C beträgt;
Cannflavin A, dargestellt durch eine Formel: 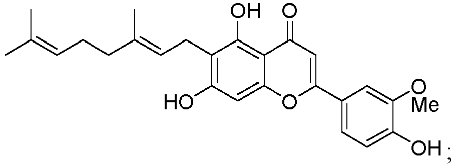
Cannflavin C, dargestellt durch eine Formel:

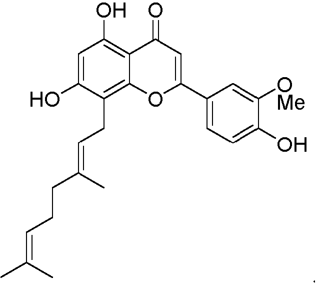

## Revendications

1. Procédé de préparation d'un composé de cannflavine, comprenant les étapes suivantes :
étape (1) : la condensation de la 4'-hydroxy-3'-méthoxyacétophénone et du carbonate de diéthyle dans des conditions alcalines pour obtenir le 4'-hydroxy-3'-méthoxybenzoylacétate d'éthyle ;
étape (2) : la soumission du 1,3,5-trihydroxybenzène et du géraniol à une réaction de C-alkylation pour obtenir le (*E*)-2-(3,7-diméthyloct-2,6-dién-1-yl)benzène-1,3,5-triphénol ; et
étape (3) : la condensation du 4'-hydroxy-3'-méthoxybenzoylacétate d'éthyle et du (*E*)-2-(3,7-diméthyloct-2,6-dién-1-yl)benzène-1,3,5-triphénol à haute température pour produire de la cannflavine A et/ou de la cannflavine C ; la température de réaction à l'étape (3) est de 180 à 230 °C ;
la cannflavine A représentée par une formule : 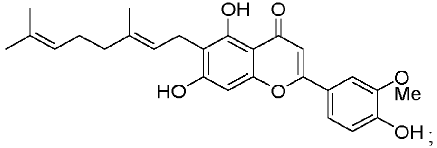
la cannflavine C représentée par une formule :

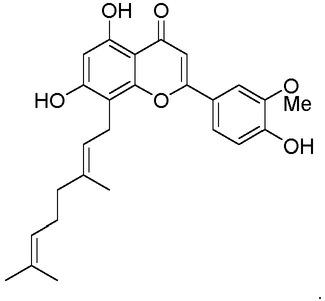

2. Procédé de préparation selon la revendication 1, dans lequel l'étape (1) comprend une étape de réaction de la 4'-hydroxy-3'-méthoxyacétophénone avec le carbonate de diéthyle ; la réaction est réalisée dans un environnement exempt d'oxygène ; la réaction est réalisée dans des conditions alcalines ; le système de réaction comprend en outre un solvant choisi dans le groupe constitué par : le toluène et le benzène.

3. Procédé de préparation selon la revendication 2, dans lequel l'environnement exempt d'oxygène à l'étape (1) est un environnement de réaction protégé par un gaz inerte ;
l'alcali est un alcali inorganique ;
un rapport molaire massique entre l'alcali inorganique et la 4'-hydroxy-3'-méthoxyacétophénone est de 50 à 1 000 g/mol ;
un rapport molaire entre la 4'-hydroxy-3'-méthoxyacétophénone et le carbonate de diéthyle est de 1:3 à 3:1 ;
la température de réaction est de 80 à 130 °C ;
le temps de réaction est de 2 à 12 h ; et
la 4'-hydroxy-3'-méthoxyacétophénone est ajoutée goutte à goutte au carbonate de diéthyle.

4. Procédé de préparation selon la revendication 1, dans lequel l'étape (1) comprend les étapes suivantes : sous protection de gaz inerte, l'ajout de NaH et de toluène dans un récipient de réaction, l'agitation, l'ajout de carbonate de diéthyle, le chauffage, l'ajout goutte à goutte d'un mélange de 4'-hydroxy-3'-méthoxyacétophénone et de toluène, l'agitation, la réaction, puis la réalisation d'un post-traitement sur le liquide de réaction résultant pour obtenir le 4'-hydroxy-3'-méthoxybenzoylacétate d'éthyle ;
le post-traitement comprend une étape de trempage ;
l'étape de trempage comprend : l'ajout d'un acide au récipient de réaction pour ajuster le pH du mélange à 4,0-7,0 ;
l'acide est un acide inorganique, et l'acide est un ou un mélange de plusieurs acides choisis dans le groupe constitué par : un acide inorganique pur d'acide acétique et une solution aqueuse de celui-ci.

5. Procédé de préparation selon la revendication 1, dans lequel l'étape (2) comprend une étape de soumission du 1,3,5-trihydroxybenzène et du géraniol à la réaction de C-alkylation, dans lequel la réaction est réalisée dans un environnement exempt d'oxygène ; et le système de réaction comprend en outre un solvant choisi dans le groupe constitué par : l'acétonitrile et l'éther diéthylique.

6. Procédé de préparation selon la revendication 5, dans lequel l'environnement exempt d'oxygène à l'étape (2) est un environnement de réaction protégé par un gaz inerte, le gaz inerte est l'azote ;
un rapport molaire entre le 1,3,5-trihydroxybenzène et le géraniol est de 1:3 à 3:1 ;
la température de réaction est de 0 à 25 °C ;
le temps de réaction est de 8 à 24 h ; et
la réaction nécessite en outre l'ajout goutte à goutte d'une solution de trifluorure de bore dans de l'éther diéthylique.

7. Procédé de préparation selon la revendication 1, dans lequel l'étape (2) comprend les étapes suivantes : l'ajout d'acétonitrile saturé de nitrate d'argent dans un récipient de réaction, puis l'ajout de 1,3,5-trihydroxybenzène et de géraniol, le refroidissement sous protection de gaz inerte, l'ajout goutte à goutte d'une solution de trifluorure de bore dans l'éther diéthylique, l'agitation, la réaction, puis la réalisation d'un post-traitement sur le liquide de réaction résultant pour obtenir le (*E*)-2-(3,7-diméthyloct-2,6-dién-1-yl)benzène-1,3,5-triphénol ;
le post-traitement comprend une étape de trempage ;
l'étape de trempage comprend : l'ajout d'un solvant de trempage au récipient de réaction ;
dans lequel le solvant de trempage est un ou plusieurs solvants choisis dans le groupe constitué par : un mélange glace-eau, l'éther *tert*-butylique de méthyle, l'éther diéthylique, l'acétate d'éthyle et l'acétate de butyle.

8. Procédé de préparation selon la revendication 1, dans lequel l'étape (3) comprend une étape de réaction du 4'-hydroxy-3'-méthoxybenzoylacétate d'éthyle avec le (*E*)-2-(3,7-diméthyloct-2,6-dién-1-yl)benzène-1,3,5-triphénol, dans lequel la réaction est réalisée dans un environnement exempt d'oxygène.

9. Procédé de préparation selon la revendication 8, dans lequel l'environnement exempt d'oxygène à l'étape (3) est un environnement de réaction protégé par un gaz inerte ;
le temps de réaction est de 2 à 8 h ; et
un rapport molaire entre le 4'-hydroxy-3'-méthoxybenzoylacétated'éthyle et le (*E*)-2-(3,7-diméthyloct-2,6-dién-1-yl)benzène-1,3,5-triphénol est de 1:3 à 3:1.

10. Procédé de préparation selon la revendication 1, dans lequel l'étape (3) comprend les étapes suivantes : sous protection de gaz inerte, l'ajout de 4'-hydroxy-3'-méthoxybenzoylacétate d'éthyle et de (*E*)-2-(3,7-diméthyloct-2,6-dién-1-yl)benzène-1,3,5-triphénol dans un récipient de réaction, l'agitation, le chauffage, la réaction, puis la réalisation d'un post-traitement sur le liquide de réaction résultant pour obtenir de la cannflavine A et/ou de la cannflavine C.

11. Procédé de préparation selon la revendication 1, comprenant les étapes suivantes :
la condensation de la 4'-hydroxy-3'-méthoxyacétophénone et du carbonate de diéthyle dans des conditions alcalines pour obtenir le 4'-hydroxy-3'-méthoxybenzoylacétate d'éthyle ;
l'ajout d'acétonitrile saturé de nitrate d'argent dans un récipient de réaction, puis l'ajout de 1,3,5-trihydroxybenzène et de géraniol, le refroidissement sous protection de gaz inerte, l'ajout goutte à goutte d'une solution de trifluorure de bore dans l'éther diéthylique, l'agitation, la réaction, puis la réalisation d'un post-traitement sur le liquide de réaction résultant pour obtenir le (*E*)-2-(3,7-diméthyloct-2,6-dién-1-yl)benzène-1,3,5-triphénol ;
sous protection de gaz inerte, l'ajout de 4'-hydroxy-3'-méthoxybenzoylacétate d'éthyle et de (*E*)-2-(3,7-diméthyloct-2,6-dién-1-yl)benzène-1,3,5-triphénol dans un récipient de réaction, l'agitation, le chauffage, la réaction, puis la réalisation d'un post-traitement sur le liquide de réaction résultant pour obtenir de la cannflavine A et/ou de la cannflavine C.

12. Utilisation de 4'-hydroxy-3'-méthoxybenzoylacétate d'éthyle dans la préparation d'un composé, ledit composé étant la cannflavine A et/ou la cannflavine C ;
dans laquelle le 4'-hydroxy-3'-méthoxybenzoylacétate d'éthyle est mis en réaction avec le (*E*)-2-(3,7-diméthyloct-2,6-dién-1-yl)benzène-1,3,5-triphénol à haute température ;
la température de réaction est de 180 à 230 °C ;
la cannflavine A représentée par une formule : 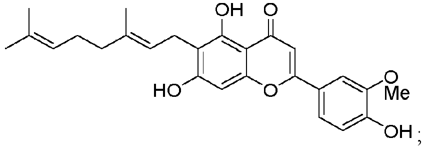
la cannflavine C représentée par une formule :
